# EUROPEAN PATENT APPLICATION

(11) **EP 4 567 097 A1**
(43) Date of publication of application: **11.06.2025**
(21) Application number: 23214282.8
(22) Date of filing: 05.12.2023
(51) Int. Cl.: C12N 1/14, C12N 1/38, C12N 3/00

(54) **CULTIVATION METHODS WITH OVERLAY**

(71) Applicant: Novozymes A/S, 2880 Bagsvaerd (DK)
(72) Inventor: ROTHSCHILD-MANCINELLI, Kyle, 2880 Bagsvaerd (DK); DAL, Aske, Bille, 2880 Bagsvaerd (DK); RAGAS, Paula, Cornelia, 2880 Bagsvaerd (DK)
(74) Representative: NVS EPO Representatives

(57) **Abstract**

The present invention relates to the use of an overlay in methods for cultivating microorganisms.

## Description

### FIELD OF THE INVENTION

The present invention relates to the use of an overlay in methods for cultivating microorganisms.

### BACKGROUND OF THE INVENTION

Microorganisms, such as bacteria and fungi, are widely used for producing enzymes and other biologicals for a variety of industrial applications.

To identify industrially relevant microorganisms, high-throughput methods represent a valuable tool by enabling, *e.g.,* faster screening and handling of the microorganisms. High-throughput methods are, however, limited when it comes to handling microorganisms with sporulating and/or filamentous morphologies. For example, hyphae tend to clog pipet tips of liquid handlers, mycelial mats make it difficult to access colonies, and automated picking and inoculation of single filamentous microbial transformants on agar plates is not as routine as for non-sporulating/non-filamentous yeast and bacteria.

A possible way of improving handling of sporulating and/or filamentous microorganisms in high-throughput settings is by controlling morphology of the microorganisms, such as, e.g., by limiting the sporulating and filamentous behaviour of the microorganisms during cultivation, so that the microorganisms become easier to work with in an automated setting.

The aim of the present invention is, therefore, to provide improved methods for cultivation of microorganisms, in particular microorganisms with sporulating and/or filamentous morphology.

### SUMMARY OF THE INVENTION

The present invention is based on the surprising and inventive finding that the morphology of a microorganism, in particular a sporulating and/or filamentous microorganism, can be controlled during cultivation of the microorganism by using an overlay with a detergent composition. For sporulating and/or filamentous microorganisms, the use of an overlay method according to the invention enables the cultivation and isolation of single colonies having non-sporulating and/or non-filamentous morphologies.

Furthermore, the morphological control obtained by the overlay is reversible such that when the microorganism is cultivated without the presence of overlay, the microorganism retains its ability to sporulate and/or form the mycelia characteristic of a filamentous morphology.

The methods of the invention thus find applicability in a wide range of applications wherein isolation of single colonies or isolates is desired, such as, *e.g.,* screening and isolation of single clones or isolates from, *e.g.,* microbiome samples and microbial strain libraries. In particular, the methods of the invention are suitable for use in high-throughput and/or automated settings..

Therefore, in a first aspect, the present invention relates to a cultivation method comprising the steps of:
(a) obtaining a cellular material on a support;
(b) overlaying the cellular material of step (a) with a detergent composition to obtain an overlaid cellular material; and
(c) incubating the overlaid cellular material under conditions suitable for cultivation, wherein the cellular material is derived or obtained from a microorganism selected from the list of a bacterium or a fungus.

In a second aspect, the present invention relates to the use of an overlay comprising a detergent composition to obtain a fungal or bacterial microorganism with a non-filamentous and/or non-sporulating morphology.

### BRIEF DESCRIPTION OF THE DRAWINGS

**Figure 1** illustrates pictures taken of *Aspergillus oryzae* cultivated with (B) or without (A) an overlay.
**Figure 2** illustrates the reversible morphology phenomenon of single colonies of *Aspergillus oryzae* obtained using the overlay method of the invention and subsequently cultivated in a liquid medium without an overlay.
**Figure 3** illustrates corresponding microscopic images of single colonies of *Aspergillus oryzae* isolated from the plates or wells shown in Figures 1 and 2, respectively.
**Figure 4** illustrates pictures taken of an environmental sample subjected to the overlay method of the invention and the corresponding isolation of single isolates therefrom.

### DEFINITIONS

In accordance with this detailed description, the following definitions apply. Note that the singular forms "a," "an," and "the" include plural references unless the context clearly indicates otherwise.

Unless defined otherwise or clearly indicated by context, all percentages are percentage by weight (percent w/w or "% (w/w)"). The term "% (w/v)" refers to the concentration of a substance in a solution, expressed as a percentage by weight per unit volume.

Reference to "about" a value or parameter herein includes aspects that are directed to that value or parameter per se. For example, description referring to "about X" includes the aspect "X". When used in combination with measured values, "about" includes a range that encompasses at least the uncertainty associated with the method of measuring the particular value and can include a range of plus or minus two standard deviations around the stated value.

The pictures featured in the figures are for the purpose of illustrating certain convenient embodiments of the invention and are not to be considered as limitation thereto.

Furthermore, it will be appreciated that embodiments described in connection with one of the aspects described herein may equally be applied to the other aspects, unless otherwise stated.

Unless defined otherwise or clearly indicated by context, all technical and scientific terms used herein have the same meaning as commonly understood by one of ordinary skill in the art to which this invention belongs.

**Cellular material:** The term "cellular material" means any material comprising living cells of a microorganism. The cellular material may for example be in the form of a single cell isolate, a mixture of cells, or a suspension comprising cells. A single cell isolate may be a single colony picked from an agar plate. A mixture of cells may be a mixture of cells from the same or different microorganisms, such as, *e.g.,* a mixture of spores and vegetative cells derived from the same or different microorganisms. A suspension comprising cells may be an aqueous suspension of cells. The suspension of cells may be derived from the same or different microorganisms. Also contemplated is a suspension of cells in the form of a dilution of cells obtained, *e.g.,* from a serial dilution of a suspension comprising cells.

The cellular material may be in the form of vegetative cells, spores, protoplasts, spheroplast, mycelia, and mixtures thereof. The cellular material may originate from a single strain or from a mixture of microorganisms. A mixture of microorganisms or cells may for example be obtained or derived from an environmental sample or a microbiome sample. In the context of the invention, an "environmental sample" is a sample in liquid or solid form obtained from the environment. Examples of environmental samples include, but are not limited to, soil samples, aquatic samples, air samples, waste samples, sediment samples and biological samples. In an embodiment, the environmental sample is a soil sample. Also contemplated is a sample taken from a microbiome. In the context of the present invention, a "microbiome sample" means a sample comprising a community of microorganisms, such as bacteria, fungi, and viruses, that inhabit a particular environment. The particular environment inhabited by the microbiome is not particularly limiting to the invention. I.e. the microbiome may be sampled from any environment. In some embodiments, the microbiome sample is obtained from a human, an animal, from wastes, such as faeces or excrements of humans or animals, or from surfaces or equipment, such as hospital equipment. Some non-limiting examples of microbiome niches include, but are not limited to, gut, skin, eye, bronchus, oral, *e.g.,* saliva or upper respiratory tract, urogenital tract, and vaginal tissue.

**Colony:** The term "colony", referred to in connection with "clone" or "isolate" or "cell", means a visible cluster or grouping of microbial cells. The colony may be cultivated on and obtained from a semi-solid or solid growth medium, such as an agar-containing growth medium. In the context of the invention, the colony may, *e.g.,* be obtained from a wild-type microorganism or from a transformant.

**Conditions essentially free of an overlay:** In the context of the invention, the term "conditions essentially free of an overlay" means conditions suitable for ensuring viability, optionally growth, of the microorganism, *e.g.,* by allowing the microorganism to grow in or on a suitable medium, wherein the condition does not include a step of overlaying with the detergent composition according to the invention.

**Conditions suitable for cultivation:** In the methods of the invention, the overlaid cellular material is incubated at conditions suitable for cultivation, meaning that the cellular material is grown under conditions suitable for viability and propagation of the microorganism. Conditions suitable for growth comprise a range of different parameters, such as, *e.g.,* temperature, exposure to light, oxygen, carbon dioxide, humidity, choice of growth medium, exposure to agitation and pH, which may be varied depending on the microbial species intended for cultivation. A person of skill in the art will know how to determine the best suitable conditions for cultivation of a given cellular material.

**Detergent:** The term "detergent" is defined herein to mean chemicals typically used in laboratory research for example as mild surfactants (surface acting agents), for cell lysis (i.e., the disruption of cell membranes) and for the release of intracellular materials. Detergents are amphiphilic molecules, containing both hydrophilic and hydrophobic regions.

Common detergents are categorized into four groups based on their characteristics: chaotropic, ionic (anionic or cationic), non-ionic and zwitterionic. Examples of detergents include, but are not limited to, sodium dodecyl sulfate (SDS), deoxycholate, cholate, sarkosyl, 3-[(3-cholamidopropyl)dimethylammonio]-1-propanesulfonate (CHAPS), urea, sodium taurocholate (NaT), polyethylene glycol sorbitan monolaurate (Tween^{®} 20), polyoxyethylenesorbitan monopalmitate (Tween^{®} 40), polyethylene glycol sorbitan monostearate (Tween^{®} 60), polyethylene glycol sorbitan monooleate (Tween^{®} 80), *t*-octylphenoxypolyethoxyethanol (Triton^{™} X-100), (1,1,3,3-Tetramethylbutyl)phenyl-polyethylene glycol (Triton^{™} X-114), Nonidet P-40 (NP-40), Octylphenoxy poly(ethyleneoxy)ethanol, branched (Igepal^{®} CA-630), Polyoxyethylene lauryl ether (Brij^{™} 35), n-dodecyl-β-D-maltoside (DDM), Digitonin and combinations thereof.

**Detergent composition:** The term "detergent composition" refers to compositions comprising at least one detergent and/or surfactant. The detergent composition may comprise further constituents, such as, *e.g.,* water, a growth promoting component, a buffering agent, or the like. A person of skill in the art of laboratory research will know of suitable constituents to include in the detergent composition depending on the application of the detergent composition.

**Essentially free of:** In the context of the invention, the term "essentially free of" is used to describe a composition, a product, or a process that contains only trace amounts or negligible quantities of a particular substance, component, or process feature. It indicates that the presence of the specified substance or process feature is minimal and does not impact the overall characteristics or functionality of the invention. In an embodiment, "essentially free of" means 0% (w/w) or 0% (w/v).

**Filamentous microorganism:** The term "filamentous microorganism" means a microorganism that grows in a thread or filamentous form, *e.g.,* by forming mycelia. The term "filamentous" may refer to the growth pattern of the microorganism, wherein the individual cells are arranged in filaments or threads. As used herein, a filamentous microorganism may be a sporulating or a non-sporulating microorganism, meaning that it may or may not be able to form spores in addition to its ability to form mycelia. In the methods of the present invention, the filamentous microbial cell may be a wild-type cell or a mutant cell. In an embodiment, the filamentous microbial cell is obtained from an environmental sample or a microbiome sample.

**Humidity:** The term "humidity" has its normal meaning which is well known and understood by those of skill in the art. It means the amount of water vapor present in the air or another gas and is a measure of the moisture content in the air. In the context of the invention, humidity is expressed as a percentage (%) indicating the amount of moisture in the air relative to the maximum amount the air could hold at its current temperature.

**Incubation:** The term "incubation" has its normal meaning which is well known and understood by those of skill in the art. It means a process of maintaining specific conditions, such as temperature, light, time, and humidity, to support the propagation or viability of living microorganisms or processes. In the context of the invention, the incubation is carried out at conditions suitable for cultivation of microbial cells which are essentially free of sporulating and/or filamentous morphology. The incubation is carried out for a time of 3-100 hours or until visible colonies appear on the support. In some embodiment, incubation is carried out for at least 12 hours, such as at least 24 hours, at least 36 hours, at least 48 hours, at least 72 hours, at least 96 hours, at least 120 hours or at least 144 hours.

**Introduced:** The term "introduced" in the context of inserting a nucleic acid sequence into a cell, means "transfection", "transformation" or "transduction," as known in the art.

**Isolation:** In the context of the invention, the term "isolation" as used to describe the process of isolating a cellular material or a cell means a process of separating or removing a single cell from a larger group of cells or a growth medium. There are several methods for isolating cells, and the choice of method depends, *e.g.,* on cell type and objectives of the application. For example, the isolation may be carried out by using a pipette tip or a toothpick to pick a single colony from, *e.g.,* an agar plate or from a well of a multi well system.

**Medium:** The terms "medium" or "growth medium" or "culture medium" are used interchangeable and have their normal meaning which is well known and understood by those of skill in the art. The medium may be a liquid, semi-solid, or solid medium designed to support the growth of a population of microorganisms or cells. Different types of media may be used for growing different types of cells and microorganisms. A person of skill in the art will know how to determine the best suitable medium depending on aim, microorganism and application.

**Microorganism with controlled morphology:** In the context of the invention, the term "microorganism with controlled morphology" means a microorganism which, when grown under conditions suitable for cultivation, is essentially free of a sporulating and/or filamentous morphology.

**Morphology:** The term "morphology" has its normal meaning which is well known and understood by those of skill in the art, and means the visual features of the microorganism, such as size, shape, and structure of the microorganisms. The morphology may be examined visually with or without the use of equipment. For example, morphology of the microorganisms may be examined using a microscope.

**Mutant:** The terms "mutant" and "variant" are used interchangeably and mean the resulting microbial strain after one or more disruptions are made to a parent microbial strain.

**Mycelium:** The term "mycelium" has its normal meaning which is well known and understood by those of skill in the art and means a root-like structure of a filamentous microorganism consisting of a mass of interwoven filamentous hyphae that forms especially the vegetative portion the microorganism.

**Overlay:** In the context of the invention, the term "overlay" means the act of laying or spreading over or across a detergent composition on a cellular material on a support. The overlay comprises the detergent composition and, thus, when used as the noun "overlay" is held to comprise at least the detergent composition. The overlay may or may not comprise further components. Preferably, the overlay is in a liquid form.

**Parent:** The term "parent" or "parent microbial strain" means a strain to which a disruption is made to produce a mutant strain described herein. The parent may be a naturally occurring (wild type) or previously modified microbial strain.

**Prewet:** The term "prewet" means the step of adding to the support, prior to or essentially at the same time as the step of obtaining the cellular material on the support, a prewetting agent. In the context of the invention, the prewet may be performed seconds, minutes or hours before obtaining the cellular material on the support.

**Protoplast and spheroplast:** The terms "protoplast" or "spheroplast" are used interchangeably and mean a cell from which the cell wall has been removed, leaving only the cell membrane and its contents. The process of removing the cell wall may be referred to as protoplast or spheroplast isolation. In the context of the invention, protoplasts or spheroplast can be derived or obtained from bacterial cells or fungal cells.

**Recombinant:** The term "recombinant" is used in its conventional meaning to refer to the manipulation, *e.g.,* cutting and re-joining, of nucleic acid sequences to form constellations different from those found in nature. The term recombinant refers to a cell, nucleic acid, polypeptide or vector that has been modified from its native state. Thus, for example, recombinant cells express genes that are not found within the native (non-recombinant) form of the cell, or express native genes at different levels or under different conditions than found in nature. The term "recombinant" is synonymous with "genetically modified" and "transgenic".

**Reversible morphology:** The term "reversible morphology" means that the changes observed in morphology when subjecting a microorganism to the methods of the invention can be reversed. I.e. a microorganism obtained using the method of the invention retains its sporulating and/or filamentous morphology when grown under conditions essentially free of the overlay. In particular, the change in morphology observed for the microorganism when overlaid with the detergent composition, such as the non-sporulating and/or non-filamentous morphology observed in the presence of the overlay, is temporary and reversible without changing the genome of the organism.

**Spore:** The terms "spore" and "endospore" are used interchangeable and have their normal meaning which is well known and understood by those of skill in the art. As used herein, the term spore refers to a microorganism in its dormant, protected state.

**Sporulating microorganism:** The term "sporulating microorganism" means a microorganism that is able to form spores. As used herein, a sporulating microorganism may be a filamentous or non-filamentous microorganism, meaning that it may or may not be able to form mycelia in addition to its ability to sporulate. In the methods of the present invention, the sporulating microbial cell may be a wild-type cell or a mutant cell. In an embodiment, the sporulating microbial cell is obtained from an environmental sample or a microbiome sample.

**Support:** The term "support" means a structure or material facilitating the maintenance of viability of the cellular material. For example, the support may be a liquid, a semi-solid, or a solid medium. The support may be held in a single-well or multi-well system, such as, *e.g.,* a support held in a petri dish or in a multi-well plate. In an embodiment, the support is an agar-containing medium formed in a petri dish or a multi-well plate.

**Surfactant:** The term "surfactant" as used herein refers to any conventional understanding of a surfactant within the art and may be referred to as a substance which reduces the surface tension of a liquid in which it is dissolved. The surfactant may be anionic and/or non-ionic and/or semi-polar and/or zwitterionic (also known as amphoteric) and/or cationic, or a mixture thereof.

**Transformant:** The term "transformant" means a cell which has taken up extracellular DNA (foreign, artificial or modified) and expresses the gene(s) contained therein. The transformant may be obtained by the process of transformation. The term "transformation" refers to a permanent or transient genetic change induced in a cell following introduction of new nucleic acid. Genetic change ("modification") can be accomplished either by incorporation of the new DNA into the genome of the host strain, or by transient or stable maintenance of the new DNA as an episomal element. Transformation may be used to produce a microbial strain having a desirable trait enabling, *e.g.,* the use of the microbial strain for production of desired proteins, such as biologicals or enzymes. The transformation may be carried out using an expression vector, a phage, a virus, or other DNA construct, or pure DNA. The term "microbial strain" includes protoplasts created from cells.

**Wild-type:** The term "wild-type" strain means a naturally occurring microorganism, such as a bacterium, yeast, or filamentous fungus found in nature or held in a microbial cell bank.

### DETAILED DESCRIPTION OF THE INVENTION

The present invention is based on the surprising and inventive finding that the morphology and growth of a microorganism, in particular a microorganism having a sporulating and/or filamentous morphology, can be controlled by subjecting a microorganism to an overlay comprising a detergent composition during cultivation of the microorganism. In particular, the inventors of the present invention have found that by using an overlay comprising a detergent composition during incubation of the microorganism at conditions suitable for cultivation, the sporulating and/or filamentous morphology of the microorganism is limited in such a way that the microorganism is essentially non-sporulating and/or non-filamentous, but still viable. Interestingly, the change in morphology is reversible, *i.e.,* when self-same microorganism, after having been subjected to the overlay method of the invention, is subsequently cultivated under conditions not comprising the overlay, the microorganism retains its sporulating and/or filamentous morphology. Thus, the methods of the invention can be used in a wide range of applications, in particular applications requiring controlled cultivation of microorganisms, optionally with the aim of isolation of single viable colonies.

The methods of the invention are particularly suitable for use in high-throughput and/or automated settings. For example, when applied in an automated high-throughput method, such as, *e.g.,* a high-throughput screening method or high-throughput genomic DNA extraction method carried out using robotic equipment (see Example 1), the control of morphology, in particular the inhibition of sporulating and/or filamentous morphological behaviour of the microorganism, provides for a lower risk of carry-over or cross-over contamination, *e.g.,* arising from picking and transferring colonies from one medium to a new, and further results in an improved chance of picking single colonies. Thus, the methods of the invention may be incorporated in high-throughput settings to increase the success rate of the high-throughput method by reducing the risk of contamination.

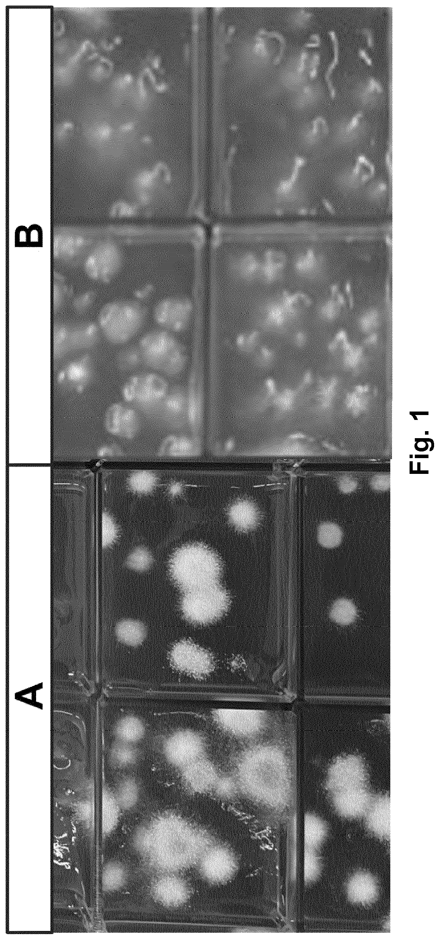

The surprising findings reported herein are exemplified in Examples 1 and 2 and illustrated in at least Figures 1 to 4, and based on these general observations, the inventors expect that the same benefits can be obtained using the method of the invention on any suitable microorganism and using any suitable detergent composition for the overlay.

In a first aspect, the present invention relates to a cultivation method comprising the steps of:
(a) obtaining a cellular material on a support;
(b) overlaying the cellular material of step (a) with a detergent composition to obtain an overlaid cellular material; and
(c) incubating the overlaid cellular material under conditions suitable for cultivation, wherein the cellular material is derived or obtained from a microorganism selected from the list of a bacterium or a fungus.

In the methods of the invention, the step of obtaining the cellular material on a support may comprise laying or spreading over the cellular material on or across the support.

In some embodiments, the conditions suitable for cultivation comprises a temperature in the range of 15°C-45°C, such as in the range of 25°C-40°C, a humidity of at least 70%, such as at least 85%, and no agitation or light exposure.

In preferred embodiments, the microorganism is a filamentous and/or sporulating microorganism.

In an embodiment, the microorganism is a filamentous fungus.

"Fungus" as used herein includes all filamentous forms found in the phyla Ascomycota, Basidiomycota, Chytridiomycota, Eumycota, and Zygomycota as well as the Oomycota and all mitosporic fungi (as defined by Hawksworth et al., In, Ainsworth and Bisby's Dictionary of The Fungi, 8th edition, 1995, CAB International, University Press, Cambridge, UK). Filamentous fungi are generally characterized by a mycelial wall composed of chitin, cellulose, glucan, chitosan, mannan, and other complex polysaccharides. Vegetative growth is by hyphal elongation and carbon catabolism is obligately aerobic. In contrast, vegetative growth by yeasts such as *Saccharomyces cerevisiae* is by budding of a unicellular thallus and carbon catabolism may be fermentative.

In some embodiments, the fungus is selected from *Acremonium, Aspergillus, Aureobasidium, Bjerkandera, Ceriporiopsis, Chrysosporium, Coprinus, Coriolus, Cryptococcus, Filibasidium, Fusarium, Gibberella, Humicola, Hyphoderma, Irpex, Junghuhnia, Lenzites, Leptoporus, Magnaporthe, Meruliopsis, Mucor, Myceliophthora, Mycoacia, Myrothecium, Neocallimastix, Neurospora, Paecilomyces, Penicillium, Phanerochaete, Phlebia, Piromyces, Pleurotus, Polyporus, Schizophyllum, Stachybotrys, Talaromyces, Thermoascus, Thielavia, Tolypocladium, Trametes, Trichoderma* or *Trichothecium.*

In some embodiments, the fungus is selected from *Aspergillus aculeatus, Aspergillus awamori, Aspergillus carbonarius, Aspergillus foetidus, Aspergillus fumigatus, Aspergillus japonicus, Aspergillus nidulans, Aspergillus niger, Aspergillus oryzae, Bjerkandera adusta, Ceriporiopsis aneirina, Ceriporiopsis caregiea, Ceriporiopsis gilvescens, Ceriporiopsis pannocinta, Ceriporiopsis rivulosa, Ceriporiopsis subrufa, Ceriporiopsis subvermispora, Chrysosporium inops, Chrysosporium keratinophilum, Chrysosporium lucknowense, Chrysosporium merdarium, Chrysosporium pannicola, Chrysosporium queenslandicum, Chrysosporium tropicum, Chrysosporium zonatum, Coprinus cinereus, Coriolus hirsutus, Fusarium bactridioides, Fusarium cerealis, Fusarium crookwellense, Fusarium culmorum, Fusarium graminearum, Fusarium graminum, Fusarium heterosporum, Fusarium negundi, Fusarium oxysporum, Fusarium reticulatum, Fusarium roseum, Fusarium sambucinum, Fusarium sarcochroum, Fusarium sporotrichioides, Fusarium sulphureum, Fusarium torulosum, Fusarium trichothecioides, Fusarium venenatum, Lenzites styracinus, Humicola insolens, Humicola lanuginosa, Hyphoderma roseocremeum, Hyphoderma radulum, Hyphoderma setigerum, Irpex latemarginatus, Junghuhnia collabens, Leptoporus mollis, Meruliopsis corium, Meruliopsis taxicola, Mucor miehei, Myceliophthora thermophila, Mycoacia fuscoatra, Neurospora crassa, Penicillium camemberti, Penicillium purpurogenum, Penicillium roqueforti, Penicillium commune, Phanerochaete chrysosporium, Phanerochaete laevis, Phanerochaete livescens, Phanerochaete sordida, Phanerochaete velutina, Phlebia lilascens, Phlebia ochraceofulva, Phlebia radiata, Pleurotus eryngii, Polyporus ciliatus, Polyporus melanopus, Polyporus umbellatus, Talaromyces emersonii, Thielavia terrestris, Trametes villosa, Trametes versicolor, Trichoderma harzianum, Trichoderma koningii, Trichoderma longibrachiatum, Trichoderma reesei, or Trichoderma viride.*

In some embodiments, the fungus is selected from *Aspergillus, Fusarium, Penicillium* and *Trichoderma.*

In some embodiments, the fungus is *Aspergillus niger, Aspergillus oryzae* or *Trichoderma reesei.*

In an embodiment, the microorganism is a sporulating bacterium, optionally a sporulating and filamentous bacterium.

In some embodiments, the microorganism is a bacterium belonging to the phyla of *Actinobacteria, Chloroflexi* or *Firmicutes.* The phylum *Firmicutes* may also be referred to as *Bacillota.*

In some embodiments, the microorganism is a bacterium selected from *Bacillus, Clostridium, Geobacillus, Oceanobacillus*, or *Streptomyces.* In an embodiment, the microorganism is selected from *Bacillus alkalophilus, Bacillus amyloliquefaciens, Bacillus brevis, Bacillus circulans, Bacillus clausii, Bacillus coagulans, Bacillus firmus, Bacillus lautus, Bacillus lentus, Bacillus licheniformis, Bacillus megaterium, Bacillus pumilus, Bacillus stearothermophilus, Bacillus subtilis, Bacillus thuringiensis, Streptomyces achromogenes, Streptomyces avermitilis, Streptomyces coelicolor, Streptomyces griseus,* and *Streptomyces lividans.*

For purposes of this invention, *Bacillus* classes/genera/species may be defined as described in Patel and Gupta, 2020, Int. J. Syst. Evol. Microbiol. 70: 406-438.

In an embodiment, the microorganism is a bacterium selected from the group of *Bacillus amyloliquefaciens, Bacillus licheniformis* and *Bacillus subtilis.*

In an embodiment, the microorganism is a bacterium selected from the group of *Streptomyces achromogenes, Streptomyces avermitilis, Streptomyces coelicolor, Streptomyces griseus* and *Streptomyces lividans.*

In the methods of the invention, the cellular material may be obtained from or derived from any living cellular material obtainable or derivable from a bacterial or fungal microorganism. For example, the cellular material may be obtained from a mutant or wild-type strain, from a cell bank sample, from an environmental sample, from a microbiome sample, or combinations thereof.

The cellular material may be derived or obtained from microorganisms isolated from the environment, such as, *e.g.,* from soil, composts, water, etc., or DNA samples obtained directly from natural materials, such as, *e.g.,* from soil, composts, water, etc. Techniques for isolating microorganisms and DNA directly from natural habitats are well known in the art.

Also, the cellular material may be derived or obtained from a mutant strain. The mutant strain may be a transformant cell.

Methods for introducing DNA into microbial cells are well-known in the art, and any suitable method can be used including, but not limited to, protoplast transformation, competent cell transformation, electroporation, conjugation, transduction, with DNA introduced as linearized or as circular polynucleotide. A person of skill in the art will be readily capable of identify a suitable method for introducing DNA into a given microbial cell depending, *e.g.,* on the genus of microorganism. Methods for introducing DNA into microbial cells are for example described in Heinze et al., 2018, BMC Microbiology 18:56, Burke et al., 2001, Proc. Natl. Acad. Sci. USA 98: 6289-6294, Choi et al., 2006, J. Microbiol. Methods 64: 391-397, and Donald, Guédon and Renault, 2013, Journal of Bacteriology, 195:11(2612-2620).

Fungal cells may be transformed by a process involving protoplast-mediated transformation, Agrobacterium-mediated transformation, electroporation, biolistic methods, and shock-wave-mediated transformation as reviewed by Li et al., 2017, Microbial Cell Factories 16: 168 and procedures described in EP 238023, Yelton et al., 1984, Proc. Natl. Acad. Sci. USA 81: 1470-1474, Christensen et al., 1988, Bio/Technology 6: 1419-1422, and Lubertozzi and Keasling, 2009, Biotechn. Advances 27: 53-75. However, any method known in the art for introducing DNA into a fungal host cell can be used, and the DNA can be introduced as linearized or as circular polynucleotide.

In an embodiment, the cellular material is selected from the list of a vegetative cell, a spore, a mycelium, a protoplast, a spheroplast, and mixtures thereof. In an embodiment, the cellular material is a protoplast or a spheroplast. In a preferred embodiment, the cellular material is in a suspension. For example, the cellular material may be a spore suspension, a mycelial suspension, a protoplast suspension, a spheroplast suspension, a vegetative cell suspension, or a suspension comprising a mixture of any of a spore, mycelium, protoplast, spheroplast, and vegetative cell.

In the context of the invention, the cellular material may be derived or obtained from a single microbial strain or from mixtures of microbial strains. Thus, when referring, *e.g.,* to a spore or a spore suspension, this is held to comprise either a spore or a spore suspension obtained from a single microbial strain or spores or a spore suspension obtained from a mixture of microbial strains. The same applies to vegetative cells, mycelia, protoplasts, and spheroplasts.

In an embodiment, the cellular material is a suspension comprising one or more spores, mycelia, vegetative cells, and mixtures thereof, wherein the cellular material is obtained or derived from a cell bank sample, an environmental sample, or a microbiome sample.

In an embodiment, the cellular material is a suspension of vegetative cells or spores obtained from a mutant strain or a wild-type strain.

In an embodiment, the cellular material is a suspension of protoplasts or spheroplasts.

In some embodiments, the overlay covers at least the cellular material. Preferably, the overlay is a complete overlay meaning that the entire surface of the cellular material in contact with the overlay is covered by the overlay. In some embodiments, the overlay covers the cellular material and the support. Also, the overlay of the cellular material and the support may be a complete overlay, *i.e.,* covering the surfaces of both the cellular material and the support in direct contact with the overlay.

In the methods of the invention, the overlay comprises a detergent composition. Preferably, the detergent composition is a liquid detergent composition. In an embodiment, the liquid detergent composition comprises sterile water or a buffering solution. An example of a buffer solution suitable for use with the methods of the invention include, but is not limited to, an STC buffer solution or a similar buffer solution.

The detergent composition may comprise one or more detergents and/or surfactants. The detergent or surfactant for use in the present invention is not particularly limited, and is selected from detergents and surfactants known in the art. The detergent and/or surfactant may be selected from the list of chaotropic detergents and/or surfactants, ionic detergents and/or surfactants, non-ionic detergents and/or surfactants, zwitterionic detergents and/or surfactants, and mixtures thereof. The person of skill in the art knows of suitable detergents and surfactants to apply in the methods of the invention.

In an embodiment, the detergent composition comprises one or more chaotropic detergents and/or surfactants. Examples of chaotropic detergents or surfactants include, but are not limited to, guanidinium chloride, guanidine hydrochloride, guanidinium thiocyanate, thiourea and urea.

In an embodiment, the detergent composition comprises one or more ionic detergents and/or surfactants. In the context of the invention, ionic detergents and/or surfactants include both anionic detergents and/or surfactants and cationic detergents and/or surfactants. Examples of ionic detergents or surfactants include, but are not limited to, sodium dodecyl sulfate (SDS), deoxycholate, cholate, sarkosyl.

In an embodiment, the detergent composition comprises one or more non-ionic detergents and/or surfactants. Examples of non-ionic detergents and surfactants include, but are not limited to, sodium taurocholate (NaT), polyethylene glycol sorbitan monolaurate (Tween^{®} 20), polyoxyethylenesorbitan monopalmitate (Tween^{®} 40), polyethylene glycol sorbitan monostearate (Tween^{®} 60), polyethylene glycol sorbitan monooleate (Tween^{®} 80), polyoxyethylenesorbitan monopalmitate (Tween^{®} 85), t-octylphenoxypolyethoxyethanol (Triton^{™} X-100), (1,1,3,3-Tetramethylbutyl)phenyl-polyethylene glycol (Triton^{™} X-114), Nonidet P-40 (NP-40), Octylphenoxy poly(ethyleneoxy)ethanol, branched (Igepal^{®} CA-630), Polyoxyethylene lauryl ether (Brij^{™} 35), n-dodecyl-β-D-maltoside (DDM), Digitonin.

In an embodiment, the detergent composition comprises one or more of polyethylene glycol sorbitan monooleate (Tween^{®} 80) and octylphenoxypolyethoxyethanol (Triton^{™} X-100). In an embodiment, the detergent composition comprises as only detergent polyethylene glycol sorbitan monooleate (Tween^{®} 80).

In an embodiment, the detergent composition comprises one or more zwitterionic detergents and/or surfactants. An example of a zwitterionic detergent include, but is not limited, to 3-[(3-cholamidopropyl)dimethylammonio]-1-propanesulfonate (CHAPS).

In some embodiments, the concentration of detergent and/or surfactant in the detergent composition is in the range of 0.01-10% (w/v). In further embodiments, the concentration of detergent and/or surfactant in the detergent composition is in the range of 0.1-8% (w/v), 0.5-6% (w/v), 1-5% (w/v), or 2-4% (w/v). In an embodiment, the concentration of detergent and/or surfactant in the detergent composition is in the range of 0.05-5% (w/v). In another embodiment, the concentration of detergent and/or surfactant in the detergent composition is about 0.1%, 1%, 2%, 3%, 4% or 5% (w/v).

In some preferred embodiments, the detergent composition is an STC buffer solution having a concentration of polyethylene glycol sorbitan monooleate (Tween^{®} 80) in the range of 0.05-6% (w/v), such as in the range of 0.1-5% (w/v).

In an embodiment, during the incubation, humidity is kept at at least 70%, such as at least 75%, at least 80%, at least 85%, at least 90%, or at least 95%. In preferred embodiments, the humidity during the incubation is kept at at least 85%.

In an embodiment, the support is a liquid, a semi-solid or a solid support. In preferred embodiments, the support is an agar-containing medium. A person of skill in the art will know of suitable agar-containing media to use with the method of the invention, for example based on the intended application of the method and the microorganism being cultivated. In some embodiments, the pH of the support is in the range of 3-10, such as in the range of 4-9, 4.5-8, or 5-7. In some embodiments, the pH of the support is about 4, about 4.5, about 5, about 5.5, about 6, about 6.5, about 7, about 7.5, or about 8.

In the methods of the invention, the support is held in a receptacle. The receptacle may be any receptacle deemed useful by a person of skill in the art, and may, *e.g.,* be a culture flask, a single-well system, such as, *e.g.,* a petri dish, or a multi well system, such as, *e.g.,* a multi well plate. In an embodiment, the support is held in a petri dish. In another embodiment, the support is held in multi-well plate.

In some embodiments, the support is an agar-containing medium formed in a single-well plate or a multi-well plate. The multi well plate may be, *e.g.,* a 6-, 12-, 24-, 36-, 48-, or 96-well plate. In a preferred embodiment, the support is an agar-containing medium held in a 48-well plate.

The support may or may not comprise a detergent and/or surfactant.

In an embodiment, the support is essentially free of a detergent and/or surfactant.

In another embodiment, the support comprises a detergent and/or surfactant. The detergent and/or surfactant comprised in the support may be any detergent and/or surfactant deemed useful by the person of skill in the art, including, but not limited to, those disclosed herein.

In an embodiment, the support comprises the same detergent(s) as used for the detergent composition. Without the wish of being bound by any particular theory, the inventors have found that by using the methods of the invention and additionally adding a detergent and/or surfactant to the support, a further improvement in separation and isolation of single colonies may be obtained due to smaller colonies forming on the support during incubation.

In an embodiment, in step (a), the support is prewet with a prewetting agent. In a preferred embodiment, the prewet is carried out prior to the step of obtaining the cellular material on the support. In some embodiments, the prewet is carried out immediately, such as within 60 seconds, prior to the step of obtaining the cellular material on the support. In some embodiments, the prewet is carried out minutes or hours prior to the step of obtaining the cellular material on the support. The prewetting agent used for the prewet is preferably in a liquid form. The prewet may be performed using essentially the same method as that of overlaying. I.e. the prewetting agent may be laid or spread over or across the support. In an embodiment, the prewet is performed using an aqueous solution, such as, *e.g.,* a buffer solution. Any buffer solution deemed suitable by the person of skill in the art may be used as prewetting agent and may for example be an STC buffer. In an embodiment, the prewetting agent further comprises a detergent and/or surfactant.

Without the wish of being bound by any particular theory, the inventors have found that the addition of a prewetting step to the methods of the invention results in an improved separation of single clones on the support. This is believed to be due to a reduced risk of the support drying out during the step of obtaining the cellular material on the support. This further improves the method of the invention, in particular when used in a high-throughput and automated setting, wherein the method of the invention would be carried out over several rounds. Also, the additional improvement in separation of single clones further reduces the risk of carry-over or cross contamination.

In an embodiment, the method of the invention further comprises the steps of:
(d) after the step of incubating the overlaid cellular material under conditions suitable for cultivation, obtaining a cellular material having a non-filamentous and/or non-sporulating morphology; and
(e) optionally isolating the cellular material having the non-filamentous and/or non-sporulating morphology.

The cellular material cultivated and optionally isolated using the methods of the invention retains its ability to form a sporulating and/or filamentous morphology when grown under conditions essentially free of the overlay of the invention. Thus, the methods of the invention are suitable for use in a wide range of applications, in particular in methods for preparing strain libraries. Also, the cellular material isolated from the methods of the invention may be further processed. In some embodiment, the cellular material obtained according to the methods of the invention is used for genomic DNA extraction. In some embodiment, the cellular material obtained according to the methods of the invention is used for obtaining single colonies for cell banks or strain libraries.

In a second aspect, the present invention relates to the use of an overlay comprising a detergent composition to obtain a fungal or bacterial microorganism with a non-filamentous and/or non-sporulating morphology.

### LIST OF EMBODIMENTS

The invention is further defined by the following numbered embodiments:
[1] Cultivation method comprising the steps of:
   (a) obtaining a cellular material on a support;
   (b) overlaying the cellular material of step (a) with a detergent composition to obtain an overlaid cellular material; and
   (c) incubating the overlaid cellular material under conditions suitable for cultivation, wherein the cellular material is derived or obtained from a microorganism selected from the list of a bacterium or a fungus.
[2] Method of embodiment 1, wherein the microorganism is a filamentous and/or sporulating microorganism.
[3] Method of any of the preceding embodiments, wherein the microorganism is a fungus.
[4] Method of any of the preceding embodiments, wherein the microorganism is a filamentous fungus.
[5] Method of any of the preceding embodiments, wherein the microorganism is selected from the list of *Acremonium, Aspergillus, Aureobasidium, Bjerkandera, Ceriporiopsis, Chrysosporium, Coprinus, Coriolus, Cryptococcus, Filibasidium, Fusarium, Gibberella, Humicola, Hyphoderma, Irpex, Junghuhnia, Lenzites, Leptoporus, Magnaporthe, Meruliopsis, Mucor, Myceliophthora, Mycoacia, Myrothecium, Neocallimastix, Neurospora, Paecilomyces, Penicillium, Phanerochaete, Phlebia, Piromyces, Pleurotus, Polyporus, Schizophyllum, Stachybotrys, Talaromyces, Thermoascus, Thielavia, Tolypocladium, Trametes, Trichoderma* or *Trichothecium.*
[6] Method of any of the preceding embodiments, wherein the microorganism is a fungus selected from *Aspergillus, Fusarium, Penicillium* and *Trichoderma.*
[7] Method of any of the preceding embodiments, wherein the microorganism is *Aspergillus niger, Aspergillus oryzae* or *Trichoderma reesei.*
[8] Method of any of embodiments 1 or 2, wherein the microorganism is a bacterium, preferably a sporulating and/or filamentous bacterium.
[9] Method of any of embodiments 1 or 2, wherein the microorganism is a bacterium selected from *Bacillus, Clostridium, Geobacillus*, *Oceanobacillus*, or *Streptomyces.*
[10] Method of any of embodiments 1 or 2, wherein the microorganism is a bacterium selected from *Bacillus alkalophilus, Bacillus amyloliquefaciens, Bacillus brevis, Bacillus circulans, Bacillus clausii, Bacillus coagulans, Bacillus firmus, Bacillus lautus, Bacillus lentus, Bacillus licheniformis, Bacillus megaterium, Bacillus pumilus, Bacillus stearothermophilus, Bacillus subtilis, Bacillus thuringiensis, Streptomyces achromogenes, Streptomyces avermitilis, Streptomyces coelicolor, Streptomyces griseus,* and *Streptomyces lividans.*
[11] Method of any of embodiments 1 or 2, wherein the microorganism is a *Bacillus amyloliquefaciens, Bacillus licheniformis* or *Bacillus subtilis.*
[12] Method of any of embodiments 1 or 2, wherein the microorganism is *Streptomyces achromogenes, Streptomyces avermitilis, Streptomyces coelicolor, Streptomyces griseus,* or *Streptomyces lividans.*
[13] Method of any of the preceding embodiments, further comprising the step of:
   (d) after step (c), obtaining a cellular material having a non-filamentous and/or non-sporulating morphology.
[14] Method of embodiment 13, further comprising the step of:
   (e) isolating the cellular material of step (d).
[15] Method of any of the preceding embodiments, wherein the cellular material is obtained from or derived from any living cellular material.
[16] Method of any of the preceding embodiments, wherein the cellular material is obtained or derived from a mutant or wild-type microbial strain, from a cell bank sample, from an environmental sample, or from a microbiome sample.
[17] Method of any of the preceding embodiments, wherein the cellular material is obtained or derived from a transformant cell.
[18] Method of any of embodiments 1-16, wherein the cellular material is derived or obtained from an environmental sample or a microbiome sample.
[19] Method of any of the preceding embodiments, wherein the cellular material is selected from the list of a vegetative cell, a spore, a mycelium, a protoplast, a spheroplast, and mixtures thereof.
[20] Method of any of the preceding embodiments, wherein the cellular material is a mixture of a spore and/or a mycelium and/or a vegetative cell, optionally wherein the cellular material is derived from one or more microorganisms.
[21] Method of any of the preceding embodiments, wherein the cellular material is a protoplast or a spheroplast.
[22] Method of any of the preceding embodiments, wherein the cellular material is in a suspension, optionally wherein the suspension is an aqueous suspension.
[23] Method of any of the preceding embodiments, wherein the overlay covers at least the cellular material.
[24] Method of any of the preceding embodiments, wherein the overlay covers both the cellular material and the support.
[25] Method of any of the preceding embodiments, wherein the ratio between the overlay and the support is between 1:10 and 1:40, such as 1:12, 1:15, 1:18, 1:20, 1:22, 1:24, 1:26, 1:28, 1:30, 1:32, 1:35, or 1:38.
[26] Method of any of the preceding embodiments, wherein the ratio between the overlay and the support is between 1:20 and 1:30, such as 1:21, 1:23, 1:25, or 1:27.
[27] Method of any of the preceding embodiments, wherein the detergent composition is a liquid detergent composition, such as a liquid detergent composition comprising sterile water or a buffer solution.
[28] Method of any of the preceding embodiments, wherein the detergent composition comprises one or more detergents and/or surfactants.
[29] Method of any of the preceding embodiments, wherein the detergent composition comprises one detergent or surfactant.
[30] Method of any of the preceding embodiments, wherein the detergent composition comprises one or more detergent and/or surfactant selected from chaotropic detergents and/or surfactants, ionic detergents and/or surfactants, non-ionic detergents and/or surfactants, zwitterionic detergents and/or surfactants, and combinations thereof.
[31] Method of any of the preceding embodiments, wherein the detergent composition comprises one or more non-ionic detergents and/or surfactants.
[32] Method of any of the preceding embodiments, wherein the detergent composition comprises one or more non-ionic detergents and/or surfactants selected from sodium taurocholate (NaT), polyethylene glycol sorbitan monolaurate (Tween^{®} 20), polyoxyethylenesorbitan monopalmitate (Tween^{®} 40), polyethylene glycol sorbitan monostearate (Tween^{®} 60), polyethylene glycol sorbitan monooleate (Tween^{®} 80), polyoxyethylenesorbitan monopalmitate (Tween^{®} 85), t-octylphenoxypolyethoxyethanol (Triton^{™} X-100), (1,1,3,3-Tetramethylbutyl)phenyl-polyethylene glycol (Triton^{™} X-114), Nonidet P-40 (NP-40), Octylphenoxy poly(ethyleneoxy)ethanol, branched (Igepal^{®} CA-630), Polyoxyethylene lauryl ether (Brij^{™} 35), n-dodecyl-β-D-maltoside (DDM), Digitonin and combinations thereof.
[33] Method of any of the preceding embodiments, wherein the detergent composition comprises one or more of polyethylene glycol sorbitan monooleate (Tween^{®} 80) and octylphenoxypolyethoxyethanol (Triton^{™} X-100).
[34] Method of any of embodiments 1-30, wherein the detergent composition comprises one or more ionic detergents and/or surfactants, optionally wherein the one or more ionic detergents and/or surfactants is selected from sodium dodecyl sulfate (SDS), deoxycholate, cholate, and sarkosyl.
[35] Method of any of the preceding embodiments, wherein the detergent composition comprises one or more detergents and/or surfactants at a concentration in the range of 0.01-10% (w/v), such as 0.1-8% (w/v), 0.5-6% (w/v), 1-5% (w/v), or 2-4% (w/v).
[36] Method of any of the preceding embodiments, wherein the detergent composition comprises one or more detergents and/or surfactants at a concentration in the range of 0.05-5% (w/v).
[37] Method of any of the preceding embodiments, wherein the detergent composition comprises one or more detergents and/or surfactants at a concentration of about 0.1%, 1%, 2%, 3%, 4% or 5% (w/v).
[38] Method of any of the preceding embodiments, wherein during the incubation, humidity is kept at at least 70%, such as at least 75%, at least 80%, at least 85%, at least 90%, or at least 95%.
[39] Method of any of the preceding embodiments, wherein during the incubation, humidity is kept at at least 85%.
[40] Method of any of the preceding embodiments, wherein during the incubation, humidity is kept at at least 90%.
[41] Method of any of the preceding embodiments, wherein the support is a liquid, a semi-solid, or a solid support.
[42] Method of any of the preceding embodiments, wherein the support is a semi-solid or a solid support.
[43] Method of any of the preceding embodiments, wherein the support comprises agar.
[44] Method of any of the preceding embodiments, wherein the support is held in a receptacle, optionally wherein the receptacle is a culture flask, a single well system, such as a petri dish, or a multi well system, such as a multi well plate.
[45] Method of any of the preceding embodiments, wherein the support is held in a single-well plate or a multi-well plate.
[46] Method of any of the preceding embodiments, wherein the support is held in a 6-well plate, a 12-well plate, a 24-well plate, a 36-well plate, a 48-well plate, or a 96-well plate, preferably a 48-well plate.
[47] Method of any of the preceding embodiments, wherein the support comprises a detergent and/or surfactant.
[48] Method of embodiment 47, wherein the detergent and/or surfactant is the same as the detergent and/or surfactant in the detergent composition.
[49] Method of any of embodiments 1-46, wherein the support is essentially free of a detergent and/or surfactant.
[50] Method of any of the preceding embodiments, wherein the support is prewet with a prewetting agent prior to obtaining the cellular material on the support.
[51] Method of embodiment 50, wherein the prewetting agent is an aqueous solution, such as a buffer solution.
[52] Method of any of embodiments 50 or 51, wherein the prewetting agent further comprises one or more detergents and/or surfactants.
[53] Method of any of embodiments 50-52, wherein the ratio between the prewetting agent and the support is between 1:10 and 1:40, , such as 1:12, 1:15, 1:18, 1:20, 1:22, 1:24, 1:26, 1:28, 1:30, 1:32, 1:35, or 1:38.
[54] Method of any of embodiments 50-53, wherein the ratio between the prewetting agent and the support is between 1:20 and 1:30, such as 1:21, 1:23, 1:25, or 1:27.
[55] Method of any of the preceding embodiments, wherein the cultivation method is suitable for use in a high-throughput method, such as an automated high-throughput method.
[56] Method of any of the preceding embodiment, wherein the cellular material obtained according to a cultivation method as embodied in any of the preceding embodiments is suitable for use in a genomic DNA extraction method.
[57] Method of any of the preceding embodiments, wherein the cellular material obtained according to a cultivation method as embodied in any of the preceding embodiments retains its ability to form a sporulating and/or filamentous morphology when grown under conditions essentially free of an overlay.
[58] Use of an overlay comprising a detergent composition to obtain a fungal or bacterial microorganism with a non-filamentous and/or non-sporulating morphology.
[59] Use according to embodiment 58, wherein the fungal or bacterial microorganism has a filamentous and/or sporulating morphology when cultivated under conditions essentially free of the overlay.

The invention described and claimed herein is not to be limited in scope by the specific embodiments herein disclosed, since these embodiments are intended as illustrations of several aspects of the invention. Any equivalent embodiments are intended to be within the scope of this invention as well as combinations of one or more of the embodiments.

Various references are cited herein, the disclosures of which are incorporated by reference in their entireties. The present invention is further described by the following examples which should not be construed as limiting the scope of the invention.

### EXAMPLES

### Example 1: Controlling morphology of Aspergillus oryzae transformants

In this example, the overlay method of the invention was tested on *Aspergillus oryzae* transformants in an automated setting using Hamilton Microlab STAR Liquid Handling System.

### Transformation of A. oryzae (general procedure)

The transformation of *A. oryzae* was carried out according to standard procedure using protoplast. How the protoplasts are prepared is not particularly limiting to the invention. One example of how the protoplasts may be prepared is disclosed in WO 95/02043 A1.

For the transformation, 100 µL of *A. oryzae* protoplasts and 7.5-15.0 µL of DNA (resuspended gBlocks^{™}) were added to each well of a 96-well plate. The plate was then shaken for 30 seconds at 600 rpm followed by incubation at room temperature for 10 minutes. Following incubation, 300 µL PEG solution (40% (w/v) PEG in STC buffer solution with 200mM CaCl₂) was added to each well of the 96-well plate and the plate was shaken 2 minutes at 600 rpm, followed by incubation at room temperature for 20 minutes.

### Plating and overlay of transformants

For the plating and overlay of *A. oryzae* transformants, a Qtray plate with cover and 48 well divider was used. The 48-well Qtray plate was prepared with approximately 6 mL sucrose/10mM NaNOs agar medium per well. Prior to plating the transformants of *A. oryzae,* each of the wells in the 48-well plate was prewet by adding 250 µL of STC buffer solution (40% sucrose, 1 M Tris-HCl, and 1 M CaCl₂) having room temperature. After the prewet, 350 µL transformation mixture was added to each well of the Qtray plate. Then, 250 µL overlay solution (2.5% (w/v) Tween80 in STC buffer solution (room temperature)) was added to each well of the Qtray plate, and 1 wet kim wipes tissue was placed on top of the Qtray plate which was then placed in a zip lock plastic bag. The Qtray plate was then incubated in an incubator (NUAIRE CO₂ waterjacketed incubator with humidity control set to 95%) in the zip lock plastic bag at 37°C for 3 days until visible colonies began to appear.

For comparison, the same protocol as specified above was repeated, but without the step of overlaying with 2.5% (w/v) Tween80 in STC buffer solution.

The result from the transformation with (B) and without (A) an overlay is shown in Figure 1. From Figure 1 (Figure 1B) it is clearly seen that by adding an overlay to the transformation mixture, optionally on plates having been prewet, the resulting colonies have a controlled morphology. In particular, it was seen that by cultivating in this case *A. oryzae* with an overlay comprising a detergent composition, single clones could be obtained which had a non-sporulating and non-filamentous morphology. Without the overlay, spores and mycelial structures were clearly observed (Figure 1A) for the clones.

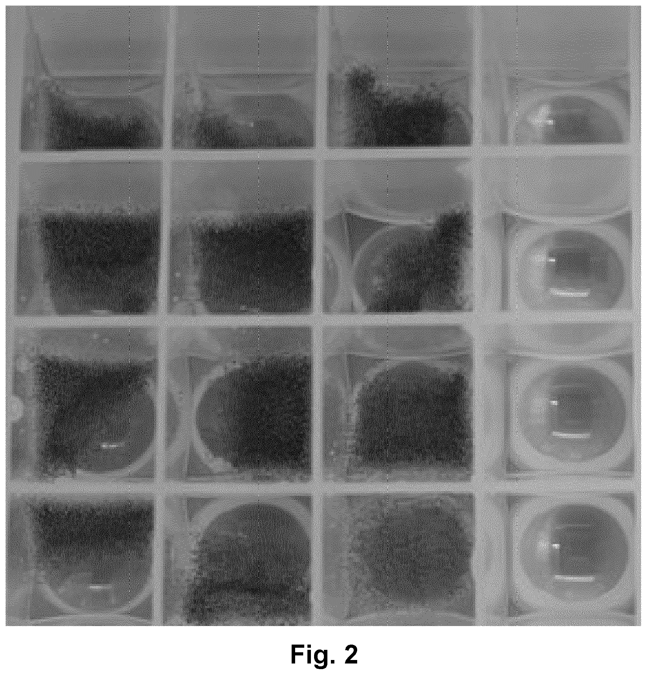

In particular, the resulting yeast-like morphology, as seen on the plate as single smooth and loose clones, enabled fast and precise picking of single clones from plates looking like those of Figure 1B. As can be seen from Figure 2, in which one (1) clone from each of the wells of the overlaid plate (Figure 1B) was grown in liquid medium, the isolated single clones retained the ability to sporulate once cultivated without the presence of the overlay. This is seen from the dark mat forming on top of the liquid medium, in particular as can seen from the black dots appearing in the first three rows from the left in the multi-well plate shown in Figure 2. The row furthest to the right in Figure 2 is blank controls, i.e. liquid medium without any cellular material added.

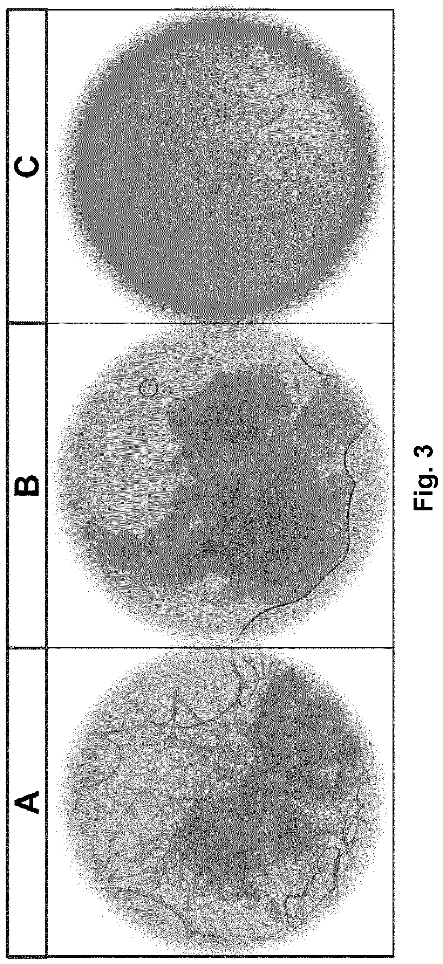

The morphological control of the microorganism, in this case *A. oryzae,* achieved using the overlay method described herein was further confirmed by examination of single clones under the microscope as illustrated in Figure 3. Here, it was seen that transformants incubated without an overlay (A) formed branched networks characteristic of a sporulating or filamentous morphology, whereas clones picked from the transformation plates with an overlay (B) resembled the morphology typical of non-sporulating or non-filamentous microorganisms. Finally, as can be seen from the picture to the right in Figure 3 (C), when an overlaid transformants was inoculated and cultivated in fresh medium not containing an overlay, the morphology of that clones was reversed to a sporulating or filamentous morphology, as evidence by the branched structure observed under the microscope.

### Example 2: Controlling morphology of a pool of microorganisms obtained from an environmental sample

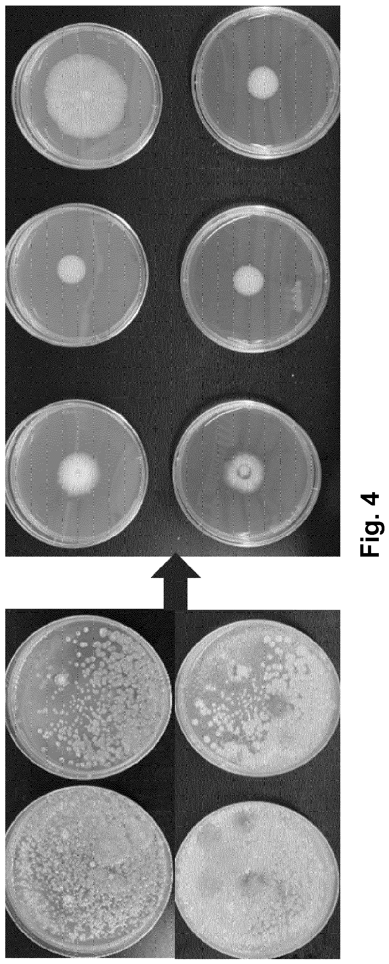

To assess the applicability of the overlay method of the invention in isolating single colonies from mixed-type samples, an experiment was carried out in which a sample taken from a compost bin was diluted in an aqueous suspension and plated in varying dilutions on agar plates. The agar plates were prepared using a similar method as that of Example 1, except the step of prewetting the agar plates was omitted. The overlay used was 2.5% (w/v) Tween80 in STC buffer solution. After 3 days incubation in an incubator set to 37°C (NUAIRE CO₂ water jacketed incubator with humidity control set to 95%), single colonies appeared on the plates (Figure 4, four agar plates to the left). From these plates, single colonies were picked and transferred onto fresh agar plates (6 agar plates to the right in Figure 4), these agar plates being prepared without the overlay. As seen from Figure 4, it was thus confirmed that single colonies of pure strains could be isolated from otherwise mixed-strain samples. In particular, the isolation could be performed with a particularly lower risk of carry-over contamination as observed by the six agar plates in Figure 4, in which only clean single colonies appeared after incubation.

To confirm that the overlay method of the invention was also applicable in construction of strain or cell databanks, genomic DNA extraction and sequencing was performed for selected single clones isolated from the overlaid plates of Example 2. As seen from Table 1, it was confirmed that even a small-scale overlay experiment resulted in the isolation of a diverse range of microorganisms.

**Table 1 Strains isolated from single sample and confirmed by sequencing.**

| **Isolate no.** | **Strain** |
|---|---|
| 1 | *Phanerochaete sordida* |
| 2 | *Meruliopsis taxicola* |
| 3 | *Phanerochaete laevis* |
| 4 | *Phanerochaete livescens* |
| 5 | *Lenzites styracinus* |
| 6 | *Aspergillus carbonarius* |
| 7 | *Phlebia ochraceofulva* |
| 8 | *Hyphoderma roseocremeum* |
| 9 | *Hyphoderma radulum* |
| 10 | *Leptoporus mollis* |
| 11 | *Hyphoderma setigerum* |
| 12 | *Meruliopsis corium* |
| 13 | *Phanerochaete velutina* |
| 14 | *Irpex latemarginatus* |
| 15 | *Phlebia lilascens* |
| 16 | *Polyporus ciliatus* |
| 17 | *Polyporus umbellatus* |
| 18 | *Mycoacia fuscoatra* |
| 19 | *Polyporus melanopus* |
| 20 | *Penicillium roqueforti* |
| 21 | *Penicillium commune* |
| 22 | *Junghuhnia collabens* |
| 23 | *Fusarium culmorum* |
| 24 | *Aspergillus aculeatus* |

Overall, these observations demonstrate the applicability of the overlay method in automated, high-throughput methods, both when working with mutant strain libraries (Example 1) and mixed-strain samples (Example 2).

## Claims

1. Cultivation method comprising the steps of:
(a) obtaining a cellular material on a support;
(b) overlaying the cellular material of step (a) with a detergent composition to obtain an overlaid cellular material; and
(c) incubating the overlaid cellular material under conditions suitable for cultivation, wherein the cellular material is derived or obtained from a microorganism selected from the list of a bacterium or a fungus.

2. Method of claim 1, wherein the microorganism is a filamentous and/or sporulating microorganism.

3. Method of any of the preceding claims, further comprising the steps of:
(d) after step (c), obtaining a cellular material having a non-filamentous and/or non-sporulating morphology; and
(e) optionally, isolating the cellular material of step (d).

4. Method of any of the preceding claims, wherein the cellular material is any living cellular material, optionally wherein the cellular material is selected from the list of a vegetative cell, a spore, a mycelium, a protoplast, a spheroplast, and mixtures thereof.

5. Method of any of the preceding claims, wherein the overlay covers at least the cellular material, optionally wherein the overlay covers both the cellular material and the support.

6. Method of any of the preceding claims, wherein the detergent composition comprises one or more detergents and/or surfactants, optionally wherein the detergent and/or surfactant is selected from chaotropic detergents and/or surfactants, ionic detergents and/or surfactants, non-ionic detergents and/or surfactants, zwitterionic detergents and/or surfactants, and combinations thereof.

7. Method of any of the preceding claims, wherein the detergent composition comprises one or more non-ionic detergents and/or surfactants, optionally wherein the one or more non-ionic detergents and/or surfactants is selected from sodium taurocholate (NaT), polyethylene glycol sorbitan monolaurate (Tween^{®} 20), polyoxyethylenesorbitan monopalmitate (Tween^{®} 40), polyethylene glycol sorbitan monostearate (Tween^{®} 60), polyethylene glycol sorbitan monooleate (Tween^{®} 80), polyoxyethylenesorbitan monopalmitate (Tween^{®} 85), *t-*octylphenoxypolyethoxyethanol (Triton^{™} X-100), (1,1,3,3-Tetramethylbutyl)phenyl-polyethylene glycol (Triton^{™} X-114), Nonidet P-40 (NP-40), Octylphenoxy poly(ethyleneoxy)ethanol, branched (Igepal^{®} CA-630), Polyoxyethylene lauryl ether (Brij^{™} 35), n-dodecyl-β-D-maltoside (DDM), Digitonin, and combinations thereof.

8. Method of any of claims 1-6, wherein the detergent composition comprises one or more ionic detergents and/or surfactants, optionally wherein the one or more ionic detergents and/or surfactants is selected from sodium dodecyl sulfate (SDS), deoxycholate, cholate, sarkosyl, and combinations thereof.

9. Method of any of the preceding claims, wherein the detergent composition comprises one or more detergents and/or surfactants at a concentration in the range of 0.01-10% (w/v), such as 0.1-8% (w/v), 0.5-6% (w/v), 1-5% (w/v), or 2-4% (w/v).

10. Method of any of the preceding claims, wherein during the incubation, humidity is kept at at least 70%, such as at least 75%, at least 80%, at least 85%, at least 90%, or at least 95%.

11. Method of any of the preceding claims, wherein the support is a liquid, a semi-solid or a solid support, preferably a semi-solid or solid support, optionally wherein the support comprises agar.

12. Method of any of the preceding claims, wherein the support is held in a single-well system or a multi-well system, optionally wherein the support is held in a single-well plate or a multi-well plate.

13. Method of any of the preceding claims, wherein the support is essentially free of a detergent and/or surfactant.

14. Method of any of the preceding claims, wherein the support is prewet with a prewetting agent prior to obtaining the cellular material on the support.

15. Use of an overlay comprising a detergent composition to obtain a fungal or bacterial microorganism with a non-filamentous and/or non-sporulating morphology.
